# EUROPEAN PATENT APPLICATION

(11) **EP 4 155 295 A1**
(43) Date of publication of application: **29.03.2023**
(21) Application number: 20935100.6
(22) Date of filing: 12.05.2020
(51) Int. Cl.: C07D 239/90, A61P 25/28, A61P 25/16, A61P 27/02

(54) **SMALL-MOLECULE COMPOUND SPAM1 FOR UP-REGULATING NEUROPEPTIDE PACAP AND RECEPTOR PAC1-R THEREOF, AND PREPARATION METHOD THEREFOR AND APPLICATION THEREOF**

(71) Applicant: Chen, Jianhuan, Guangzhou, Guangdong 510000 (CN); Yu, Rongjie, Guangzhou, Guangdong 510000 (CN)
(72) Inventor: YU, Rongjie, Guangzhou, Guangdong 510000 (CN)
(74) Representative: Cabinet Chaillot
(86) International application number: PCT/CN2020/089716
(87) International publication number: WO 2021/226814

(57) **Abstract**

A small molecule compound SPAM1 for up-regulating neuropeptide PACAP and receptor PAC1-R thereof, a method for preparing the same and use of the same; the SPAM1 has a structure of formula (I), wherein R = none, H₂O or HCl; SPAM1 has small molecular weight, and can efficiently cross biological barriers including a blood-brain barrier, a blood-testis barrier etc; SPAM1 up-regulates the expression of the neurotransmitter/neuromodulator PACAP secreted by hypothalamus-pituitary and the specific receptor thereof, and acts on the glands downstream of the gonadal axis and the adrenal axis, thereby exerting comprehensive regulation effects; and SPAM1 specifically targets the PAC1-R1, and only acts on cells and tissues of nervous and endocrine systems naturally expressing the PAC1-R, thereby producing fewer side effects. Accordingly, SPAM1 is a novel small molecule compound medicament for effectively treating and preventing function decline and disorders of nervous, endocrine and immune systems closely related to neuropeptide PACAP.

## Description

### TECHNICAL FIELD

The present invention relates to the field of chemistry and biochemistry, in particular to a small molecule compound SPAM1 (4-(4-(4-oxo-3,4-dihydroquinazolin-2-yl) butyryl)methylbenzoic acid) and a derivative thereof for effectively up-regulating the expression of a neuropeptide pituitary adenylate cyclase activating polypeptide (PACAP) and a specific receptor PAC1-R thereof; a method for preparing the SPAM1 and use of the SPAM1 in preventing and treating physiological and pathological diseases and function decline of nervous, endocrine and immune systems related to the PACAP and the specific receptor PAC1-R thereof.

### BACKGROUND

The neuropeptide PACAP, originally isolated from the hypothalamus-pituitary of cattle, belongs to the vasoactive intestinal peptide/secretin/growth hormone releasing hormone/glucagon superfamily. The PACAP is extremely conservative active peptide in the evolution process, and there is only one amino acid difference between frog PACAP and human PACAP^{[1]}. The PACAP mediates the important function of regulating nervous, endocrine and immune systems through three class B G protein-coupled receptors: one PACAP-specific receptor PAC1-R and two shared receptors of vasoactive intestinal peptide VPAC1-R and VPAC2-R. The down-regulation of PACAP as an important neurotransmitter and neuromodulator with age is considered to be one of the causes of aging.

The PACAP and a receptor, particularly PAC1-R, thereof, are distributed at high density not only on hypothalamus-pituitary, but also on neuroendocrine tissues and glands such as pituitary-adrenal axis, pituitary-gonadal axis, pineal body and thymus, including adrenal gland, testis and ovary; and mediate the secretion and regulation of related stress hormones, sex hormones and immune factors, including the up-regulation of corticotropin releasing hormone (CRH) and corticosterone (COR), the involvement in the regulation of melatonin and gonadotrophin releasing hormone (GnRH) release, the up-regulation of testosterone (TE) and estradiol (E2). In contrast, PACAP-deficient animals have declining thymus and down-regulated immunologic function. In summary, existing cell and animal studies have shown that the PACAP and the PAC1-R mediate the stress regulation of the body, enhance the sexual function and reproductive ability of male and female, promote the immunologic function and effectively reduce the death rate of sepsis.

Although the PACAP has potential anti-aging pharmaceutical development value, direct development of the PACAP into a drug is greatly limited in view of the extremely poor stability of the PACAP *in vivo,* e.g., an *in vivo* half-life of less than 2 min in PACAP38, and a limited function of crossing biological barriers.

The small molecule SPAM1 (4-(4-(4-oxo-3,4-dihydroquinazolin-2-yl)butyryl) methylbenzoic acid) is a PAC1-R small molecule allosteric modulator obtained by targeting an allosteric regulatory site located in the N-terminal extracellular domain of PAC1-R, by *in silico* virtual screening, and by cell-level and animal-level validation.

Our latest cytological and zoological studies firstly discover that the SPAM1 can up-regulate the expression of the PACAP and the PACAP-specific receptor PAC1-R in a target organ *in vivo* in a feedback-positive and concentration-dependent manner. The subsequent animal experiments of a D-galactose-induced mouse aging model firstly confirm that SPAM1 can play a role in regulating pituitary-adrenal axis, pituitary-gonadal axis and thymus by up-regulating the PACAP/PAC1-R signal pathway to resist the body decline caused by the down-regulation of the PACAP, specifically by: 1) up-regulating the stress ability of the body, 2) enhancing the sexual function and reproductive ability, and 3) resisting hypoimmunity.

### SUMMARY

The purpose of the present invention is to provide a small molecule SPAM1 that can effectively up-regulate the expression of a PACAP and a specific receptor PAC1-R thereof *in vivo.*

In order to achieve the above purpose, the present invention adopts the following technical schemes:
A small molecule compound SPAM1 for up-regulating a neuropeptide PACAP and a receptor PAC1-R thereof, having a structure of formula (I), wherein R = none or H₂O or HCl (no modification, hydration modification or hydrochlorination modification);

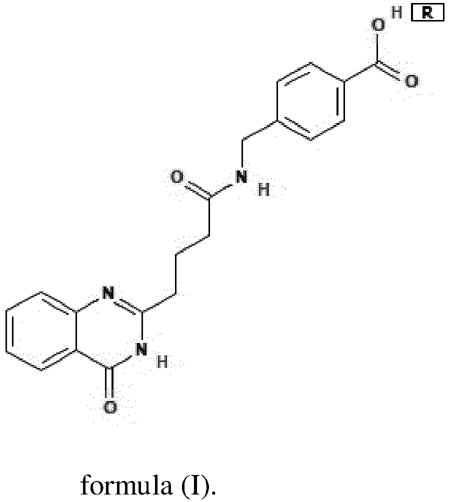

A compound SPAM1-3 having the following structures (formula (II), A-C);

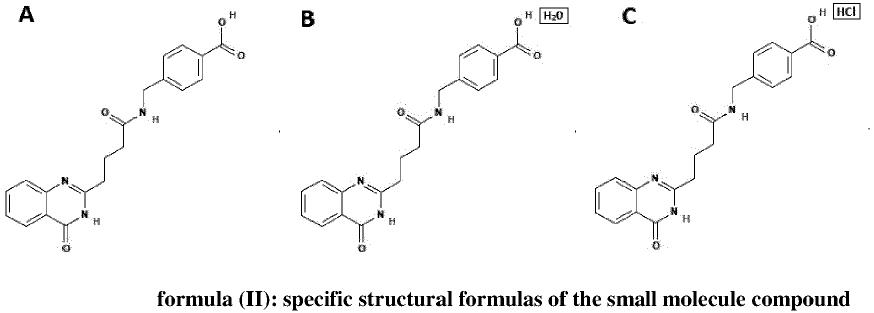

The method for preparing the small molecule compound SPAM1 is a two-step synthesis (formula (III) I, II), comprising the following steps:
(1) subjecting 2-aminobenzamide (A) and glutaric anhydride (B) to a polymerization reaction to obtain a basic compound 4-(4-oxo-3,4-dihydroquinazolin-2-yl)butyric acid (C); and
(2) subjecting the 4-(4-oxo-3,4-dihydroquinazolin-2-yl)butyric acid (C), benzo[d][1,2,3]triazol-1-ol (D), 4-(aminomethyl)benzoic acid (E) and triethylamine (F) to a condensation reaction;

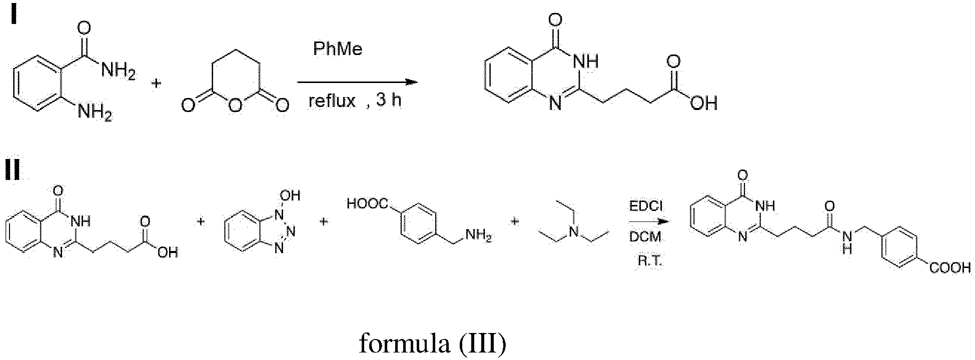

Use of the above small molecule compound SPAM1 in preparing a medicament for preventing or treating function decline and disorders of nervous endocrine or immune system closely related to the neuropeptide PACAP.

Use of the above small molecule compound SPAM1 in preparing a medicament for preventing or treating function decline and disorders of hypothalamus-pituitary-adrenal axis, hypothalamus-pituitary-gonadal axis or thymus.

Use of the above small molecule compound SPAM1 in preparing a medicament for prolonging life, preventing and treating body aging with age, male and female climacteric syndrome, male and female sexual function decline with age, male and female reproductive function decline, immunologic function disorders and decline, or sepsis.

Compared with the prior art, the present invention has the following beneficial effects:
(1) the SPAM1 of the present invention has small molecular weight and can efficiently cross biological barriers including a blood-brain barrier, a blood-testis barrier and the like;
(2) the SPAM1 of the present invention up-regulates the expression of the neurotransmitter/ neuromodulator PACAP secreted by hypothalamus-pituitary and the specific receptor PAC1-R thereof in a feedback-positive manner, and acts on the glands downstream of the gonadal axis and the adrenal axis, thereby exerting comprehensive regulation effects;
(3) the SPAM1 of the present invention specifically targets the PAC1-R1, and only acts on cells and tissues of nervous and endocrine systems naturally expressing the PAC1-R, thereby producing fewer side effects.

Accordingly, the SPAM1 will become a novel small molecule compound medicament for effectively treating and preventing function decline and disorders of nervous, endocrine and immune systems closely related to the neuropeptide PACAP.

### BRIEF DESCRIPTION OF THE DRAWINGS

FIG. 1: Synthesis process of the small molecule SPAM1 (a two-step method); A: 2-aminobenzamide; B: glutaric anhydride; C: 4-(4-oxo-3,4-dihydroquinazolin-2-yl) butanoic acid; D: benzo[d][1,2,3]triazol-1-ol; E: 4-(aminomethyl)benzoic acid; and F: triethylamine.
FIG. 2: NMR spectrum of the intermediate product C; ¹HNMR (400 MHz, DMSO): 12.16 (m, 2H), 8.08 (d, J = 5.8 Hz, 1H), 7.79-7.76 (m, 1H), 7.60 (d, J = 6.6 Hz, 1H), 7.46 (d, J = 6.0 Hz, 1H), 2.64 (t, J = 6.0 Hz, 2H), 2.32 (t, J = 5.6 Hz, 2H), 2.00-1.96 (m, 2H).
FIG. 3: NMR spectrum of the SPAM1; ¹HNMR (400 MHz, DMSO-d6) = 12.79 (s, 1H), 12.18 (s, 1H), 8.42 (t, J = 6.0 Hz, 1H), 8.09 (d, J = 7.9 Hz, 1H), 7.90 (d, J = 7.9 Hz, 2H), 7.78 (t, J, 1H), J = 7.6 Hz, 1H), 7.61 (d, J = 8.2 Hz, 1H), 7.47 (t, J = 7.5 Hz, 1H), 7.36 (d, J = 7.9 Hz, 2H), 4.33 (d, J = 5.9 Hz, 2H), 2.64 (t, J = 7.4 Hz, 2H), 2.27 (t, J = 7.5 Hz, 2H, 2.09 = 1.95 (m, 2H).
FIG. 4: The SPAM1 up-regulating the concentration of PACA in plasma *in vivo* (*, P<0.01, SPAM1+ vs. SPAM1-).
FIG. 5: The SPAM1 up-regulating the expression of PAC1-R in nerve cells Neuro2a in a concentration-dependent manner in the westernblot assay.
FIG. 6: The SPAM1 up-regulating the expression of PAC1-R in nerve cells SHSY-5Y effectively in the immunofluorescence assay.
FIG. 7: The SPAM1 injected intraperitoneally up-regulating the level of corticosterone in serum in a concentration-dependent manner in the enzyme linked immunization assay (*, P<0.01, SPAM1 vs. control group).
FIG. 8: The SPAM1 up-regulating estradiol and luteinizing hormone effectively for resisting D-galactose-induced ovarian decline (*, P<0.01, aging model group vs. normal group; #, P<0.01, SPAM1 treatment group vs. aging model group).
FIG. 9: The SPAM1 up-regulating testosterone effectively for resisting D-galactose-induced testicular decline (*, P<0.01, aging model group vs. normal group; #, P<0.01, SPAM1 treatment group vs. aging model group).
FIG. 10: The SPAM1 up-regulating thymus index effectively for resisting dexamethasone-induced thymus decline (*, P<0.01, aging model group vs. normal group; #, P<0.01, SPAM1 treatment group vs. aging model group).
FIG. 11: The SPAM1 reducing death rate of LPS-induced sepsis effectively for improving survival rate.

### DETAILED DESCRIPTION

The present invention will be further described with reference to specific examples, which are not intended to limit the present invention in any way.

### Example 1: Pilot-scale synthesis process of the SPAM1 (a two-step method)

As shown in Figure 1, a two-step synthesis was used. Step 1: In a 150 mL flask equipped with a condenser, benzamide A (5.45 g, 40.0 mmol, 1.0 eq) and amber hydride B (4.00 g, 40.0 mmol, 1.0 eq) were mixed with toluene (50 mL). The suspension was strongly refluxed for 3 h and then cooled to room temperature. The obtained product C in the form of a white solid was filtered, washed with Et₂O and dried to obtain the product (8.833 g, 88.3% yield). NMR spectrum of the product was shown in FIG. 2.

### Step 2: Synthesis according to the following feeding ratio:

| Project code | C | D | E | F | EDCI | DCM |
|---|---|---|---|---|---|---|
| Source (g) | 10.00 | 5.82 | 6.51 | 13.07 | 9.08 | 200 mL |
| Molar equivalent (eq) | 1.0 | 1.0 | 1.0 | 3.0 | 1.1 | 20 mL/g |

At room temperature, C and D were added sequentially to a 500 mL reaction flask, followed by the solvents DCM and F, and the reaction system was stirred. A shrinking agent EDCI was added in portions and the system was clarified to a dark brown color. E was added to the reaction system for further reaction until the starting material E disappeared and the reaction was completed. Central control process: a developing agent: DCM/MeOH = 3/1, sample: 1-2 drops; 1 drop of thin HCl was completely dissolved with a small amount of MeOH, and the plate was developed to control the starting material E. 24 h later, the reaction solution was directly concentrated to remove the reaction solvent, and a muddy gray crude product was obtained. The obtained muddy crude was slurried with DMF/H2O = 1/3 (4 mL/g, relative to the crude product) at room temperature with stirring overnight, and then filtered. The filter cake was washed with EtOH/EA = 1/1 to off-white and dried. The product (6.4 g, 40.6% yield) in the form of a white solid was obtained, which was SPAM1. NMR spectrum of SPAM1 was shown in FIG. 3.

### Example 2: The SPAM1 effectively up-regulating the level of PACAP in the body

Thirty BALb/c mice (SPF grade, 8-10 weeks old, weighing 22-27 g, half female and half male) were randomly divided into following 3 groups, with 10 mice in each group: 1) SPAM1 low-dose group, intraperitoneal injection, 10 µmol/kg; 2) SPAM1 high-dose group, intraperitoneal injection, 100 µmol/kg; 3) normal control group, injected with normal saline intraperitoneally; 30 min after the injection, blood was collected from the orbit, and serum was assayed for PACAP using an ELISA kit. The results were shown in FIG. 4: the SPAM1 effectively up-regulated the level of PACAP in serum and the up-regulation was concentration-dependent.

### Example 3: The SPAM1 effectively up-regulating the expression of PAC1-R-specific receptor

Mouse nerve cells Neuro2a naturally expressing the PAC1-R were cultured until the fusion rate was more than 80%, added with SPAM1 at 1 µM, 10 µM and 100 µM, and disrupted after being incubated for 1 h. The supernatant of the whole cell lysate was subjected to SDS-PAGE and the western blot assay using the anti-PAC1-R (C-terminal) antibody. The results were shown in FIG. 5: SPAM1 at 1 µM, 10 µM and 100 µM up-regulated the expression of PAC1-R in nerve cells in a concentration-dependent manner.

Human nerve cells SHSY-5Y naturally expressing the PAC1-R were cultured until the fusion rate was more than 80%, added with 100 µM SPAM1, and observed by confocal immunofluorescence after being incubated for 1 h. The results showed that the cell nucleus was stained purple blue with DAPI, and the red fluorescent signal targeting PAC1-R was significantly up-regulated by SPAM1 (FIG. 6).

### Example 4: The SPAM1 effectively up-regulating the level of corticosterone in vivo

Forty BALb/c mice (SPF grade, 8-10 weeks old, weighing 22-27 g, half female and half male) were randomly divided into following 3 groups, with 10 mice in each group: 1) SPAM1 low-dose group, intraperitoneal injection, 10 µmol/kg; 2) SPAM1 medium-dose group, intraperitoneal injection, 100 µmol/kg; 3) SPAM1 high-dose group, intraperitoneal injection, 1000 µmol/kg; 4) normal control group, injected with normal saline intraperitoneally; 1 h after the injection, blood was collected from the orbit, and the level of COR in serum was measured by the solid phase sandwich enzyme-linked immunosorbent assay (ELISA). The results were shown in FIG. 7: the SPAM1 increased the level of COR in a concentration-dependent manner, indicating that the SPAM1 increased the stress ability of the body.

### Example 5: The SPAM1 effectively resisting D-galactose-induced ovarian decline

A D-galactose-induced aging model was used. Forty BALB/c female mice (SPF grade, 8-10 weeks old, weighing 22-27 g) were randomly divided into following 4 groups, with 10 mice in each group: 1) aging model group, injected with D-galactose (200 mg/kg/d*42d) subcutaneously at the neck or back; 2) SPAM1 low-dose treatment group, injected with D-galactose as in group 1), and injected with low-dose SPAM1 (10 µmol/kg/d^{∗}28d) intraperitoneally on day 15; 3) SPAM1 high-dose treatment group, injected with D-galactose as in group 1), and injected with high-dose SPAM1 (100 µmol/kg/d^{∗}28d) intraperitoneally on day 15; and 4) normal control group, injected with normal saline intraperitoneally instead of D-galactose and SPAM1; 24 h after the completion of all injections, blood was collected from the orbit, and the level of estradiol and luteinizing hormone in serum was assayed by the enzyme-linked immunosorbent assay (ELISA). The results were shown in FIG. 8: D-galactose significantly down-regulated the level of estradiol and luteinizing hormone in serum of female mice, and the SPAM1 at low concentration and high concentration both effectively up-regulated the level of estradiol and luteinizing hormone in serum of aging mice, with the effect in the high-concentration group more significant than the low-concentration group, suggesting that the SPAM1 effectively resists ovarian decline.

### Example 6: The SPAM1 effectively resisting D-galactose-induced testicular decline

A D-galactose-induced aging model was used. Forty BALB/c male mice (SPF grade, 8-10 weeks old, weighing 22-27 g) were randomly divided into following 4 groups, with 10 mice in each group: 1) aging model group, injected with D-galactose (200 mg/kg/d*42d) subcutaneously at the neck or back; 2) SPAM1 low-dose treatment group, injected with D-galactose as in group 1), and injected with low-dose SPAM1 (10 µmol/kg/d^{∗}28d) intraperitoneally on day 15; 3) SPAM1 high-dose treatment group, injected with D-galactose as in group 1), and injected with high-dose SPAM1 (100 µmol/kg/d^{∗}28d) intraperitoneally on day 15; and 4) normal control group, injected with normal saline intraperitoneally instead of D-galactose and SPAM1; 24 h after all injections, blood was collected from the orbit, and the level of testosterone in serum was assayed by the enzyme-linked immunosorbent assay (ELISA). The results were shown in FIG. 9: D-galactose significantly down-regulated the level of testosterone in serum of male mice, and the SPAM1 at low concentration and high concentration both effectively up-regulated the level of testosterone in the serum of aging mice, with the effect in the high-concentration group more significant than the low-concentration group, suggesting that the SPAM1 effectively resists testicular decline.

### Example 7: The SPAM1 effectively resisting thymus decline

A dexamethasone-induced thymus decline model was used. Forty BALB/c mice (SPF grade, half male and half female, 8-10 weeks old, weighing 22-27 g) were randomly divided into following 4 groups, with 10 mice in each group: 1) aging model group, injected with dexamethasone (25 mg/kg/d*2d) intraperitoneally; 2) SPAM1 low-dose treatment group, injected with dexamethasone as in group 1), and injected with SPAM1 at a low dose (10 µmol/kg/d^{∗}2d) intraperitoneally at the same time; 3) SPAM1 high-dose treatment group, injected with dexamethasone as in group 1), and injected with SPAM1 at a high dose (100 µmol/kg/d^{∗}2d) intraperitoneally at the same time; and 4) normal control group, injected with normal saline intraperitoneally instead of dexamethasone and SPAM1; 24 h after the completion of the final injection, the mice were weighed and sacrificed by cervical dislocation method, the thymus of the mice was weighed, and the thymus index was calculated according to: thymus index = thymus weight (mg)/body weight (g). The results were shown in FIG. 10: the SPAM1 at low concentration and high concentration both effectively inhibited the dexamethasone-induced down-regulation of thymus index.

### Example 8: The SPAM1 effectively reducing the death rate of LPS-induced sepsis

An LPS-induced sepsis model was used. Forty BALB/c mice (SPF grade, half male and half female, 8-10 weeks old, weighing 22-27 g) were randomly divided into following 4 groups, with 10 mice in each group: 1) sepsis model group, injected with normal saline intraperitoneally, and 30 min later, injected with 15 mg/kg LPS at the tail vein; 2) SPAM1 low-dose treatment group, injected with low-dose SPAM1 (10 µmol/kg) intraperitoneally, and 30 min later, injected with 15 mg/kg LPS at the tail vein; 3) SPAM1 high-dose treatment group, injected with high-dose SPAM1 (100 µmol/kg) intraperitoneally, and 30 min later, injected with 15 mg/kg LPS at the tail vein; and 4) normal control group, injected with normal saline intraperitoneally, and 30 min later, injected with normal saline at the tail vein; the survival of mice was observed every 24 h, and the survival rate of each group was recorded for 8 days. The results were shown in FIG. 11: the SPAM1 at low concentration and high concentration both effectively inhibited death caused by LPS-induced sepsis, and reduced the death rate of sepsis.

## Claims

1. A small molecule compound SPAM1 for up-regulating a neuropeptide PACAP and a receptor PAC1-R thereof, having a structure of formula (I), wherein R = none, H₂O or HCl;

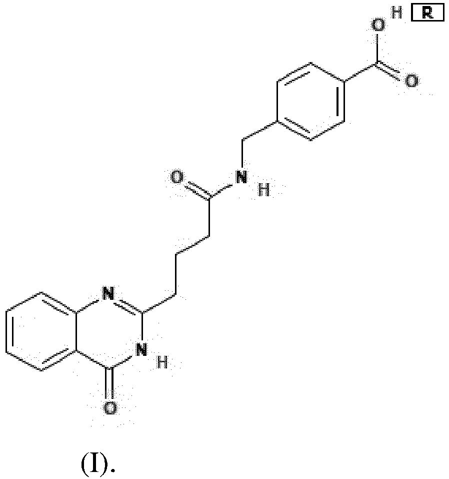

2. A method for preparing the small molecule compound SPAM1 according to claim 1, being a two-step synthesis comprising the following steps:
(1) subjecting 2-aminobenzamide and glutaric anhydride to a polymerization reaction to obtain a basic compound 4-(4-oxo-3,4-dihydroquinazolin-2-yl)
butyric acid; and
(2) subjecting the 4-(4-oxo-3,4-dihydroquinazolin-2-yl)butyric acid, benzo[d][1,2,3]triazol-1-ol, 4-(aminomethyl)benzoic acid and triethylamine to a condensation reaction.

3. Use of the small molecule compound SPAM1 according to claim 1 in the manufacture of a medicament for preventing or treating function decline and disorders of nervous, endocrine or immune system closely related to the neuropeptide PACAP.

4. Use of the small molecule compound SPAM1 according to claim 1 in the manufacture of a medicament for preventing or treating function decline and disorders of hypothalamus-pituitary-adrenal axis, hypothalamus-pituitary-gonadal axis or thymus.

5. Use of the small molecule compound SPAM1 according to claim 1 in the manufacture of a medicament for prolonging life, preventing and treating body aging with age, male and female climacteric syndrome, male and female sexual function decline with age, male and female reproductive function decline, immunologic function disorders and decline, or sepsis.
